(19) Europäisches Patentamt
European Patent Office
Office européen des brevets

(11) EP 1 083 173 B1

(12) EUROPÄISCHE PATENTSCHRIFT

(45) Veröffentlichungstag und Bekanntmachung des Hinweises auf die Patenterteilung:
**03.10.2007 Patentblatt 2007/40**

(21) Anmeldenummer: **00118649.3**

(22) Anmeldetag: **29.08.2000**

(51) Int Cl.:
***C07D 255/02*** (2006.01) ***C11D 3/395*** (2006.01)
***D06L 3/02*** (2006.01) ***D21C 1/00*** (2006.01)
***C07F 13/00*** (2006.01) ***C07F 15/02*** (2006.01)
***C07F 15/06*** (2006.01)

# (54) Bleichaktive Metallkomplexe

Metal complexes with bleaching activity

Complexes métalliques activateurs de blanchiment

(84) Benannte Vertragsstaaten:
**BE CH DE ES FR GB IT LI**

(30) Priorität: **10.09.1999 DE 19943254**

(43) Veröffentlichungstag der Anmeldung:
**14.03.2001 Patentblatt 2001/11**

(73) Patentinhaber: **Clariant Produkte (Deutschland) GmbH**
**65929 Frankfurt am Main (DE)**

(72) Erfinder:
- **Seebach, Michael, Dr.**
**65795 Hattersheim (DE)**
- **Reinhardt, Gerd, Dr.**
**65779 Kelkheim (DE)**
- **Schönherr, Thomas**
**09337 Hohenstein-Ernstthal (DE)**
- **Weber, Edwin, Prof. Dr.**
**09599 Freiberg (DE)**

(56) Entgegenhaltungen:
**EP-A- 0 544 490** **EP-A- 0 902 021**
**WO-A-96/06154**

- **DIRIL ET AL.: J. AMER. CHEM. SOC., Bd. 111, Nr. 14, 1989, Seiten 5102-5114, XP009004202**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 083 173 B1

## Beschreibung

Es ist bekannt, daß sich das Bleichvermögen peroxidischer Bleichmittel, wie Wasserstoffperoxid, Perboraten, Percarbonaten, Persilikaten und Perphosphaten, in Wasch- und Reinigungsmitteln und somit deren Effizienz bei der Entfernung von Tee-, Kaffee-, Obst- oder Rotweinflecken erst bei Temperaturen von deutlich über 60°C voll entfaltet. Zur Verbesserung der vor allem bei Temperaturen unter 60°C stark herabgesetzten Bleichwirkung können Verbindungen zur Aktivierung der Peroxidbleichmittel eingesetzt werden. Für diesen Zweck wurden eine Reihe von Übergangsmetallsalzen bzw. entsprechende Komplexe mit meist chelatisierenden Verbindungen vorgeschlagen, doch ist die Wirksamkeit eines Metalls bzw. einer speziellen Kombination von Übergangsmetall und Komplexligand nicht voraussagbar.

In den Schriften WO 96 06154 und EP 458 397 werden Metall-Komplexe mit einem hohen Aktivierungspotential beansprucht. Ein Nachteil der dort beschriebenen Komplextypen
ist, dass diese die Fasern und Farben von Textilien erheblich schädigen.
In J. Am. Chem. Soc., 1989, 111, 5102-5114 wird die Kristallstruktur eines zweikernigen Mangankomplexes mit einem verbrückenden 2,6-Bis-(1,4,7-triazacyclononan-1-yl-methyl)-4-methylphenolat-Liganden, zwei verbrückenden Acetatgruppen und zwei Perchlorat-Anionen beschrieben, wobei die Oxidationsstufen der beiden Metallzentren gemischt sind und einem Manganatom die Oxidationszahl II und dem zweiten Manganatom die Oxidationszahl III zugeschrieben wird. Über die anwendungstechnische Relevanz dieser Verbindung wurde bislang nicht berichtet.

Gegenstand der vorliegenden Erfindung sind bisher nicht bekannte Verbindungen der allgemeinen Formel I, die das Bleichvermögen von Peroxoverbindungen auch bei niedrigen Temperaturen stärken ohne die Textilfasern oder Textilfarbstoffe anzugreifen

$$[L_nM_mX_p]^zY_q \qquad \text{Formel I}$$

wobei

| | |
|---|---|
| M | für Mangan in der Oxidationsstufe II, III, IV und/ oder V, Eisen in der Oxidationsstufe II und/ oder III oder Kobalt in der Oxidationsstufe II und/ oder III steht, |
| X | eine Koordinations- oder Brückengruppe darstellt, |
| Y | ein Gegenion in der entsprechenden stöchiometrischen Menge zum Ausgleich einer vorhandenen Ladung z bedeutet, wobei |
| z | als Ladung des Metall-Komplexes positiv, null oder negativ sein kann, |
| n und m | unabhängig voneinander ganze Zahlen von 1 bis 4, |
| p | eine ganze Zahl von 0 bis 15, |
| q | z/ Ladung von Y |
| L | ein organischer Ligand der allgemeinen Formel II ist |

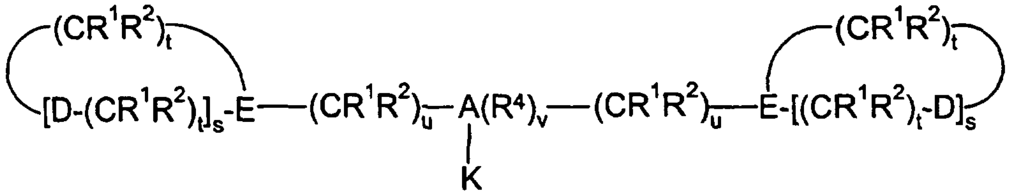

**(II)**

wobei

-$R^1$ und $R^2$ unabhängig voneinander Wasserstoff, eine Alkylgruppe mit 1 bis 6 Kohlenstoffatomen, eine Alkoxygruppe mit 1 bis 3 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, bedeuten,
-jedes D unabhängig voneinander $NR^3$, O, $PR^3$ oder S bedeutet, wobei $R^3$ für eine Alkylgruppe mit 1 bis 6 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, steht,
-jedes E unabhängig voneinander für N, P oder $C(R^1)$ steht, mit den oben für $R^1$ angegebenen Bedeutungen,
-t für eine Zahl von 0 bis 6, s für eine Zahl von 1 bis 5 und u für eine Zahl von 1 bis 4 stehen,
-A einen $C_3$- bis $C_9$-Cycloalkyl-, Phenyl-, 1,1'-Biphenyl-, Naphtyl-, Anthracenyl- oder Pyridinylrest bedeutet,
-jedes $R^4$ unabhängig voneinander Wasserstoff, eine $C_1$- bis $C_6$-Alkylgruppe, eine $C_1$- bis $C_3$-Alkoxygruppe,

eine $C_3$- bis $C_{10}$-Cycloalkylgruppe oder eine Phenylgruppe, die auch substituiert sein kann, bedeutet,
-v für eine Zahl von 0 bis 15 steht,
-K eine an mindestens eines der Metallzentren M koordinierende Gruppe vom Typ -O, -OR, -S, -SR, $-NR_2$, -NR, $-PR_2$ oder -PR bedeutet, wobei R für Wasserstoff, eine Alkylgruppe mit 1 bis 6 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, steht,

X ein Anion der folgenden Formeln bedeutet:
$F^-$, $Cl^-$, $Br^-$, $SCN^-$, $OH^-$, $O_2^{2-}$, $O^{2-}$, $O_2^-$, $R^{10}OO^-$, $H_2O$, $HS^-$, $CN^-$, $OCN^-$, $S^{2-}$, $N_3^-$, $NH_3$, $NR^{10}_3$, $NR^{10}{}_2$, $R^{10}O^-$, $R^{10}COO^-$, $R^{10}SO_3^-$ und $R^{10}SO_4^-$, wobei $R^{10}$ jeweils für Wasserstoff, $C_1$- bis $C_8$-Alkyl, $C_3$- bis $C_8$-Cycloalkyl oder $C_6$- bis $C_{18}$-Aryl steht, und

Y - bei positivem z ein Anion der folgenden Formeln: $F^-$, $Cl^-$, $Br^-$, $NO_3^-$, $ClO_4^-$, $SCN^-$, $PF_6^-$, $R^{10}SO_4^-$, $R^{10}COO^-$, $R^{10}SO_3^-$,$BF_4^-$, $BPh_4^-$ und $SO_4^{2-}$ und - bei negativem z ein Kation der folgenden Formeln: $Li^+$, $Na^+$, $K^+$, $Mg^{2+}$, $Ca^{2+}$, $Al^{3+}$, $NH_4^+$, $R^{10}NH_3^+$, $R^{10}_2NH_2^+$, $R^{10}_3NH^+$ und $R^{10}_4N^+$, wobei $R^{10}$ die zuvor genannte Bedeutung hat, bedeutet.

Bevorzugt sind Verbindungen, bei denen D für $NR^3$, E für Stickstoff, $R^1$ und $R^2$ für Wasserstoff, s für eine Zahl von 1 bis 3, t für eine Zahl von 1 bis 4 stehen, K eine Gruppe vom Typ -O, -OR, -S oder $-NR_2$ bedeutet und die übrigen Symbole die oben genannten Bedeutungen haben.

Besonders bevorzugt sind Verbindungen, bei denen A für eine Phenyl- oder $C_3$-$C_9$-Cycloalkylgruppe, m für eine Zahl von 1 bis 3 und n für eine Zahl von 1 bis 2 stehen. Soweit einzelne Symbole bei der Formel II eine substituierte Phenylgruppe bedeuten, können solche Phenylgruppen substituiert sein durch eine bis drei Gruppen der folgenden Art: $C_1$-$C_4$-Alkyl, $C_1$-$C_4$-Alkoxy, Halogenatome, substituierte oder unsubstituierte Amino- oder Ammoniumgruppen, Sulfogruppen, Carboxylgruppen oder Gruppierungen der Formel $-(CH_2)_rCOOH$, $-(CH_2)_r-SO_3H$, $-(CH_2)_r-PO_3H_2$, $-(CH_2)_l-OH$, wobei r eine ganze Zahl von 0 bis 4 und l eine ganze Zahl von 1 bis 4 bedeutet und die genannten Säuregruppen auch in Salzform vorliegen können.

Die erfindungsgemäßen Übergangsmetallkomplexe gemäß Formel I werden synthetisiert aus einem heterocyclischen verbrückenden Liganden L gemäß Formel II, z.B. 2,6-Bis(4,7-dimethyl-1,4,7-triazacyclonon-1-ylmethyl)-4-methylphenol, gelöst in einem polaren Lösemittel, vorzugsweise einem Alkohol, zu dem im Molverhältnis 1:2 eine Übergangsmetallverbindung, z.B. Mangan(II)acetat-tetrahydrat, sowie die notwendige Menge einer das Gegenion Y enthaltenden Verbindung, z. B. $KPF_6$, bei einem pH-Wert zwischen 7 und 11 in einem Temperaturbereich von 0 bis 100 °C, vorzugsweise 25 bis 50 °C, zugegeben wird. Die in den erfindungsgemäßen Komplexen enthaltenen verbrückenden Liganden der allgemeinen Formel II können beispielsweise in Analogie zu der in J. Am. Chem. Soc. 1994, 116, 22, 10334-10335 beschriebenen Synthese durch Umsetzung einer heterocyclischen Verbindung vom Typ der Formel III

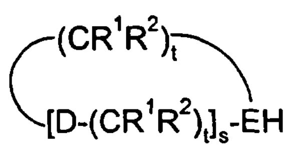

(III)

mit den oben genannten Definitionen für D, E, $R^1$, $R^2$, t und s, beispielsweise 1,4-Dimethyl-1,4,7-triazacyclononan, gelöst in einem polaren Lösungsmittel, bevorzugt einem Alkohol, in Gegenwart von äquimolaren Mengen einer Base, bevorzugt Triethylamin, mit einer Verbindung vom Typ $Z-(CR^1CR^2)_u-A(K)(R^4)_v-(CR^1CR^2)_u-Z$ mit den oben genannten Definitionen für $R^1$, $R^2$, $R^4$, A, K, u und v, wobei Z für Chlor, Brom, Jod oder eine andere Abgangsgruppe steht, im Molverhältnis 2 zu 1 im Temperaturbereich von -30 bis 80°C, bevorzugt -10 bis 50°C, im Laufe von 10 bis 120 Stunden synthetisiert werden.

Die erfindungsgemäßen mehrkernigen Komplexe der allgemeinen Formel I eignen sich hervorragend als Bleich- und Oxidationskatalysatoren, insbesondere in Wasch- und Reinigungsmitteln und bei der Textil- und Papierbleiche. Besonders hervorzuheben sind hier Textilwaschmittel in Form von Pulverwaschmitteln oder als flüssige Formulierungen und Geschirrreinigungsmittel. Ein Vorteil der erfindungsgemäßen Bleichkatalysatoren ist hierbei ihre Stabilität gegen Hydrolyse und Oxidation sowie ihre katalytische Wirkung bereits bei niedrigen Temperaturen. Sie verbessern in solchen Formulierungen nicht nur die Bleichwirkung von Wasserstoffperoxid, sondern auch von organischen und anorganischen Peroxyverbindungen.

Gegenstand der vorliegenden Erfindung ist demgemäß auch ein Verfahren zum Bleichen von verschmutzten Substraten, wobei man das verschmutzte Substrat in wässriger Bleichflotte mit einer Peroxyverbindung und einer wirksamen Menge eines oder mehrerer der erfindungsgemäßen Metall-Komplexe gemäß Formel I, wobei $R^3$ auch Wasserstoff und A auch Kohlenstoff sein kann, als Bleichkatalysatoren in Kontakt bringt.

Die erfindungsgemäßen Wasch- und Reinigungsmittelformulierungen enthalten Metall-Komplexe dieser Art in den Gewichtsmengen von 0,0001 bis 0,5 Gew.-% Metall, insbesondere 0,00025 bis 0,25 Gew.-% Metall, vor allem 0,0005 bis 0,1 Gew.-% Metall, bezogen auf das Gewicht der Formulierungen.

Diese Wasch- und Reinigungsmittel, die als pulver- oder tablettenförmige Feststoffe, homogene Lösungen oder Suspensionen vorliegen können, können außer den erfindungsgemäß eingesetzten Bleichkatalysatoren im Prinzip alle bekannten und in derartigen Mitteln üblichen Inhaltsstoffe, wie Persauerstoffverbindungen, Bleichaktivatoren, weitere konventionelle Bleichkatalysatoren, Tenside, Builder, wassermischbare organische Lösungsmittel, Enzyme, Sequestriermittel, Elektrolyte, pH-Regulatoren und weitere Hilfsmittel, wie Silberkorrosionsinhibitoren, Schaumregulatoren, Verdickungsmittel, Konservierungsmittel, Perlglanzmittel und Emulgatoren sowie Farb- und Duftstoffe enthalten.

Als geeignete Persauerstoffverbindungen kommen insbesondere organische Persäuren beziehungsweise Salze organischer Persäuren in Betracht. Beispiele hierfür sind Peroxynaphthoesäure, Peroxylaurinsäure, Peroxystearinsäure, N,N-Phthaloylamidoperoxycapronsäure, Perbenzoesäure, 1,12-Diperoxydodecandisäure, 1,9-Diperoxyazelainsäure, Diperoxysebacinsäure, Diperoxyisophthalsäure, 2-Decyldiperoxybutan-1,4-disäure, 4,4'-Sulfonyl-bisperoxybenzoesäure und Alkanoylamidoperoxycarbonsäuren.
Ebenfalls geeignet ist Wasserstoffperoxid und unter den Wasch- und Reinigungsbedingungen Wasserstoffperoxid abgebende Verbindungen wie Alkalimetallperoxide, organische Peroxide wie Harnstoff-Wasserstoffperoxid-Addukte und anorganische Persalze, wie die Alkaliperborate, -percarbonate, -perphosphate, -persilikate, -persulfate, -peroxynitrite und Hydrogenperoxocarbonat-Peroxohydrate. Besonders bevorzugt sind Natriumperborat-Tetrahydrat und insbesondere Natriumperborat-Monohydrat. Natriumperborat-Monohydrat ist wegen seiner guten Lagerbeständigkeit und seiner guten Löslichkeit in Wasser bevorzugt. Natriumpercarbonat kann aus Umweltschutzgründen bevorzugt sein. Alkylhydroperoxide sind eine weitere geeignete Gruppe von Peroxidverbindungen. Beispiele für diese Stoffe sind Cumolhydroperoxid und Butylhydroperoxid. Weiterhin eignen sich als Peroxyverbindungen anorganische Peroxysäure-Salze, z.B. Kaliummonopersulfat. Mischungen aus zwei oder mehreren dieser Verbindungen sind ebenfalls geeignet.

Die erfindungsgemäßen Wasch- und Reinigungsmittel-Formulierungen enthalten üblicherweise 1 bis 30 Gew.-%, insbesondere 2 bis 25 Gew.-% an Peroxyverbindungen.

Der Zusatz geringer Mengen bekannter Bleichmittelstabilisatoren wie beispielsweise von Phosphonaten, Boraten, Metaboraten und Metasilikaten sowie Magnesiumsalzen wie Magnesiumsulfat kann zweckdienlich sein.

Neben den Peroxyverbindungen können die Wasch- und Reinigungsmittel zusätzlich auch sogenannte Bleichaktivatoren in üblichen Mengen von ca. 1 bis 10 Gew.-% enthalten.
Als Bleichaktivatoren können Verbindungen, die unter Perhydrolysebedingungen aliphatische Percarbonsäuren mit vorzugsweise 1 bis 10 C-Atomen, insbesondere 2 bis 4-C-Atomen und/oder gegebenenfalls substituierte Perbenzoesäure ergeben, eingesetzt werden. Geeignet sind Substanzen, die O- und/ oder N-Acylgruppen der genannten C-Atomzahl und/ oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Alkanoylamidocarbonsäureester, insbesondere Alkanoylamidocapronsäurephenolsulfonate, Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran, Natrium-nonanoyloxybenzolsulfonat, Natrium-isononanoyloxy-benzolsulfonat, Natrium-4-benzoyloxy-benzolsulfonat, Natrium-trimethylhexanoyloxy-benzolsulfonat, Lactone, Acylale, Carbonsäureamide, Acyllactame, acylierte Harnstoffe und Oxamide, N-acylierte Hydantoine, beispielsweise 1-Phenyl-3-acetylhydantoin, Hydrazide, Triazole, Hydrotriazine, Urazole, Diketopiperazide, Sulfurylamide, und/ oder N-acylierte Lactame, beispielsweise N-Benzoylcaprolactam, aber auch quaternäre Nitrilverbindungen, beispielsweise quaternäre Trialkylammoniumnitrilsalze, insbesondere das Cyanomethyltrimethylammoniumsalz, aber auch heterocyclisch substituierte quaternäre Nitrilverbindungen und die aus den deutschen Patentanmeldungen DE 196 16 693 und DE 196 16 767 bekannten Enolester sowie acyliertes Sorbitol und Mannitol beziehungsweise deren in der Schrift EP 0 525 239 beschriebenen Mischungen (SORMAN), acylierte Zuckerderivate, insbesondere Pentaacetylglukose (PAG), Pentaacetylfructose, Tetraacetylxylose und Octaacetyllactose sowie acetyliertes, gegebenfalls N-alkyliertes Glucamin und Gluconolacton.
Derartige Bleichaktivatoren sind im üblichen Mengenbereich, vorzugsweise in Mengen von 1 bis 10 Gew.-%, insbesondere 2 bis 8 Gew.-%, bezogen auf das gesamte Mittel, enthalten.
Zusätzlich zu den oben aufgeführten konventionellen Bleichaktivatoren oder an deren Stelle können auch Sulfonimine und/oder weitere bleichverstärkende Übergangsmetallsalze beziehungsweise Übergangsmetallkomplexe enthalten sein.
Zu den in Frage kommenden Übergangsmetallverbindungen gehören insbesondere die aus der Schrift DE 195 29 905 und DE 196 05 688 bekannten Mangan-, Eisen-, Cobalt-, Ruthenium- oder Molybdänkomplexe.

Als oberflächenaktive Mittel können die erfindungsgemäßen Wasch- und Reinigungsmittel anionische Tenside in Mengen von ungefähr 1 bis 50 Gew.-%, bezogen auf die Gesamtmenge aller Tenside, enthalten. Bevorzugte anionische Tenside sind $C_8$-$C_{20}$-Fettsäure-alpha-methylestersulfonate, Alkylethersulfate und sekundäre Alkansulfonate.
Die in den erfindungsgemäßen Mitteln eingesetzten Alkylethersulfate sind wasserlösliche Salze oder Säuren der Formel $RO(A)_mSO_3M$, worin R einen unsubstituierten $C_{10}$-$C_{24}$-Alkyl- oder $C_{10}$-$C_{24}$-Hydroxyalkylrest, bevorzugt einen $C_{12}$-$C_{20}$-Alkyl- oder $C_{12}$-$C_{20}$-Hydroxyalkylrest, besonders bevorzugt $C_{12}$-$C_{18}$-Alkyl- oder $C_{12}$-$C_{18}$-Hydroxyalkylrest darstellt. "A" ist eine Ethoxy- oder Propoxyeinheit, m ist eine Zahl größer als 0, vorzugsweise zwischen 0,5 und ca. 6, besonders bevorzugt zwischen ca. 0,5 und ca. 3 und M ist ein Wasserstoffatom oder ein Kation wie z.B. ein Metallkation (z.B. Natrium, Kalium, Lithium, Calcium, Magnesium, etc.), Ammonium oder ein substituiertes Ammoniumkation. Spezifische Beispiele von substituierten Ammoniumkationen sind Methyl-, Dimethyl-, Trimethylammonium- und quaternäre Ammoniumkationen, wie Tetramethylammonium und Dimethylpiperidiniumkationen sowie solche, die von Alkylaminen, wie Ethylamin, Diethylamin, Triethylamin abgeleitet sind. Als Beispiele für diese Alkylethersulfate seien genannt $C_{12}$-$C_{18}$-Alkyl-polyethoxylat(1,0)sulfat ($C_{12}$-$C_{18}$E(1,0)M), $C_{12}$-$C_{18}$-Alkylpolyethoxylat(2,25)sulfat ($C_{12}$-$C_{18}$E(2,25)M), $C_{12}$-$C_{18}$-Alkyl-polyethoxylat(3,0)sulfat ($C_{12}$-$C_{18}$E(3,0)M), $C_{12}$-$C_{18}$-Alkyl-polyethoxylat(4,0)sulfat ($C_{12}$-$C_{18}$E(4,0)M).

Bei den sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe ist statistisch über die gesamte C-Kette verteilt,
wobei die primären Methylgruppen am Kettenanfang und Kettenende keine Sulfonatgruppen besitzen. Die bevorzugten sekundären Alkansulfonate enthalten lineare Alkylketten mit 9 bis 25 Kohlenstoffatomen, bevorzugt von 10 bis 20 Kohlenstoffatome und besonders bevorzugt 13 bis 17 Kohlenstoffatome. Das Kation ist Natrium, Kalium, Ammonium, Mono-, Di- oder Triethanolammonium, Calcium oder Magnesium und Mischungen davon. Natrium als Kation ist der Einfachheit halber bevorzugt.

Neben diesen oder anstelle dieser bevorzugten anionischen Tenside können die erfindungsgemäßen Formulierungen auch andere Typen von anionischen Tensiden innerhalb den oben angegebenen Grenzwerten enthalten, wie zum Beispiel Alkylsulfate, -sulfonate, -carboxylate, -phosphate und Mischungen aus den genannten Verbindungen. Geeignete Kationen sind z.B. Natrium, Kalium, Calcium oder Magnesium, sowie Ammonium, substituierte Ammoniumverbindungen, einschließlich Mono-, Di- oder Triethanolammoniumkationen, sowie Mischungen dieser Kationen. Die anionischen Tenside, die für die vorliegende Erfindung geeignet sind, weisen Tensideigenschaften auf und sind wasserlöslich oder in Wasser dispergierbar.

Alkylsulfate sind hier wasserlösliche Salze oder Säuren der Formel $ROSO_3M$, worin R bevorzugt ein $C_{10}$-$C_{24}$-Kohlenwasserstoffrest, bevorzugt ein Alkyl- oder Hydroxyalkylrest mit $C_{10}$-$C_{20}$-Alkylkomponenten, besonders bevorzugt ein $C_{12}$-$C_{18}$-Alkyl- oder Hydroxyalkylrest darstellt. M ist Wasserstoff oder ein Kation, z.B. Natrium, Kalium, Lithium oder Ammonium oder substituiertes Ammonium, z.B. Methyl-, Dimethyl- und Trimethylammoniumkationen und quaternäre Ammoniumkationen, wie Tetramethylammonium- und Dimethylpiperidiniumkationen und quartäre Ammoniumkationen, abgeleitet von Alkylaminen wie Ethylamin, Diethylamin, Triethylamin und Mischungen davon.

Ein weiteres geeignetes anionisches Tensid ist Alkylbenzolsulfonat. Die Alkylgruppe kann entweder gesättigt oder ungesättigt, verzweigt oder linear und gegebenenfalls mit einer Hydroxylgruppe substituiert sein.
Die bevorzugten Alkylbenzolsulfonate enthalten lineare Alkylketten mit 9 bis 25 Kohlenstoffatomen, bevorzugt von 10 bis 13 Kohlenstoffatomen, das Kation ist Natrium, Kalium, Ammonium, Mono-, Di- oder Triethylammonium, Calcium oder Magnesium und Mischungen davon.

Weitere geeignete anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder Alpha-Sulfonatgruppen enthalten. Bevorzugt sind lineare gesättigte oder ungesättigte Kohlenwasserstoffreste als hydrophobe Komponente in den Seifen. Üblicherweise enthalten die hydrophoben Komponenten 6 bis 30 Kohlenstoffatome, bevorzugt 10 bis 18 Kohlenstoffatome.
Weitere anionische Tenside sind Salze von Acylaminocarbonsäuren, die durch Umsetzung von Fettsäurechloriden mit Natriumsarkosinat im alkalischen Medium entstehen (Acylsarcosinate) sowie Fettsäure-Eiweiß-Kondensationsprodukte, die durch Umsetzung von Fettsäurechloriden mit Oligopeptiden erhalten werden. Tensidcharakter haben auch die Salze von Alkylsulfamidocarbonsäuren und die Salze von Alkyl- und Alkylarylethercarbonsäuren.

Andere anionische Tenside, die nützlich für den Einsatz in Reinigungsmitteln sind, sind $C_8$-$C_{24}$ Olefinsulfonate, sulfonierte Polycarboxylsäuren, hergestellt durch Sulfonierung der Pyrolyseprodukte von Erdalkalimetallcitraten, wie z.B. beschrieben in GB 1 082 179, Alkylglycerinsulfate, Fettacylglycerinsulfate, Oleylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinamate, Sulfosuccinate, Monoester der Sulfosuccinate (besonders gesättigte und ungesättigte $C_{12}$-$C_{18}$-Monoester) und Diester der Sulfosuccinate (besonders gesättigte und ungesättigte $C_{12}$-$C_{18}$-Diester), Acylsarcosinate, Sulfate von Alkylpolysacchariden wie Sulfate von Alkylglycosiden, verzweigte primäre Alkylsulfate und Alkylpolyethoxycarboxylate wie die der Formel $RO(CH_2CH_2)_kCH_2COOM$, worin R ein $C_8$-$C_{22}$-Alkyl, k eine Zahl von 0 bis 10 und M ein lösliches Salz bildendes Kation ist. Harzsäuren oder hydrierte Harzsäuren, wie Rosin oder hydriertes Rosin oder Tallölharze und Tallölharzsäuren sind ebenfalls einsetzbar. Weitere Beispiele sind in "Surface Active Agents and Detergents" (Vol. I und II, Schwartz, Perry und Berch) beschrieben. Eine Vielzahl solcher Tenside sind auch in US 3 929 678 beschrieben.

Typische Beispiele für anionische Tenside sind auch Alkylethersulfonate, Glycerinethersulfonate, Sulfofettsäuren, Fettalkoholethersulfate, Glycerinethersulfate, Hydroxymischethersulfate, Fettsäureamid-(ether)sulfate, Mono- und Dialkylsulfosuccinate, Mono- und Dialkylsulfosuccinamate, Sulfotriglyceride, Amidseifen, Alkyloligoglucosidsulfate, Alkylaminozuckersulfate und Alkyl (ether)phosphate. Sofern die anionischen Tenside Polyglycoletherketten enthalten, können sie eine konventionelle oder auch eingeengte Homologenverteilung aufweisen.

In den erfindungsgemäßen Mitteln können nichtionische Tenside, wie Fettsäurealkylesteralkoxylate, Alkyl- und/oder Alkenyloligoglykoside, Fettalkoholpolyglycolether, Alkylphenolpolyglycolether, Fettsäurepolyglykolester, Fettsäureamidpolyglycolether, Fettaminpolyglycolether, alkoxylierte Triglyceride, Fettsäureglucamide, Polyölfettsäureester, Zukkerester, Sorbitanester und Polysorbate und/oder alkoxylierte Fettalkohole eingesetzt werden. Der Anteil der nichtionischen Tenside insgesamt an der Gesamtmenge aller Tenside in den erfindungsgemäßen Reinigungsmitteln beträgt im Allgemeinen 1 bis 50 Gew.-%.

Desweiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide, besonders die Monoethanolamide aus Palmkernöl- und Kokosöl-Fettsäuren oder Polyhydroxyamide in den Gewichtsmengen 1 bis 50 % eingesetzt werden.

Der Gesamtanteil der oberflächenaktiven Verbindungen kann bis zu 50 Gew.-% betragen, vorzugsweise 1 bis 40 Gew.-%, insbesondere 4 bis 25 Gew.-% des gesamten Wasch- oder Reinigungsmittels betragen.

Als organische und anorganische Gerüststoffe (Builder) eignen sich neutral oder insbesondere alkalisch reagierende Salze, die Calciumionen ausfällen oder komplex binden können. Geeignet und insbesondere ökologisch unbedenkliche Buildersubstanzen, sind kristalline, schichtförmige Silikate der allgemeinen Formel $NaMSi_{(x)}O_{(2x+1)}$, wobei M für Natrium oder Wasserstoff, x für eine Zahl von 1,9 bis 22, vorzugsweise von 1,9 bis 4 und y für eine Zahl von 0 bis 33 steht, beispielsweise Na-SKS-5 ($\alpha$-$Na_2Si_2O_5$), Na-SKS-7 ($\beta$-$Na_2Si_2O_5$, Natrosilit), Na-SKS-9 ($NaHSi_2O_5*H_2O$), Na-SKS-10 ($NaHSi_2O_3*3H_2O$, Kanemit), Na-SKS-11 (t-$Na_2Si_2O_5$) und Na-SKS-13 ($NaHSi_2O_5$), insbesondere aber Na-SKS-6 ($\delta$-$Na_2Si_2O_5$) sowie feinkristalline, synthetische wasserhaltige Zeolithe, insbesondere vom Typ NaA, die ein Calciumbindevermögen im Bereich von 100 bis 200 mg CaO/g aufweisen. Zeolithe und Schichtsilikate können in einer Menge bis zu 20 Gew.-% im Mittel enthalten sein.

Desweiteren eignen sich nicht oder teilweise neutralisierte (co)polymere Polycarbonsäuren. Hierzu gehören die Homopolymere der Acrylsäure oder der Methacrylsäure bzw. deren Copolymere mit weiteren ethylenisch ungesättigten Monomeren wie beispielsweise Acrolein, Dimethylacrylsäure, Ethylacrylsäure, Vinylessigsäure, Allylessigsäure, Maleinsäure, Fumarsäure, Itaconsäure, (meth)allylsulfonsäure, Vinylsulfonsäure, Styrolsulfonsäure, Acrylamidomethylpropansulfonsäure sowie Phosphorgruppen enthaltende Monomere wie beispielsweise Vinylphosphorsäure, Allylphosphorsäure und Acrylamidomethylpropanphosphorsäure und deren Salze, sowie Hydroxyethyl(meth)acrylatsulfat, Allylalkoholsulfat und Allylalkoholphosphate.

Bevorzugte (Co-)Polymere weisen eine mittlere Molmasse von 1000 bis 100 000 g/mol, vorzugsweise von 2000 bis 75 000 g/mol und insbesondere von 2000 bis 35 000 g/mol auf.

Der Neutralisierungsgrad der Säuregruppen liegt vorteilhafterweise bei 0 bis 90 %, vorzugsweise bei 10 bis 80 % und insbesondere bei 30 bis 70 %.

Zu den geeigneten Polymeren zählen vor allem auch Homopolymere der Acrylsäure und Copolymere der (Meth-)Acrylsäure mit Maleinsäure bzw. Maleinsäureanhydrid.

Weitere geeignete Copolymere leiten sich von Terpolymeren ab, die sich durch Polymerisation von 10 bis 70 Gew.-% monoethylenisch ungesättigten Dicarbonsäuren mit 4 bis 8 C-Atomen, deren Salzen, 20 bis 85 Gew.-% monoethylenisch ungesättigten Monocarbonsäuren mit 3 bis 10 C-Atomen bzw. deren Salzen, 1 bis 50 Gew.-% einfach ungesättigten Monomeren, welche nach der Verseifung Hydroxylgruppen an der Polymerkette freisetzen, und 0 bis 10 Gew.-% weiteren, radikalisch copolymerisierbaren Monomeren erhalten lassen.

Ebenfalls geeignet sind Pfropfpolymerisate von Monosacchariden, Oligosacchariden, Polysacchariden und modifizierten Polysacchariden sowie tierischen oder pflanzlichen Proteinen.

Bevorzugt sind Copolymerisate aus Zucker und anderen Polyhydroxyverbindungen und einer Monomermischung aus 45 bis 96 Gew.-% monoethylenisch ungesättigten $C_3$- bis $C_{10}$-Monocarbonsäuren oder Mischungen von $C_3$- bis $C_{10}$-Monocarbonsäuren und/ oder deren Salze mit einwertigen Kationen, 4 bis 55 Gew.-% monoethylenisch ungesättigte Monosulfonsäuregruppen enthaltende Monomere, monoethylenisch ungesättigte Schwefelsäureester, Vinylphosphorsäureester und/ oder die Salze dieser Säuren mit einwertigen Kationen sowie 0 bis 30 Gew.-% wasserlösliche ungesättigte Verbindungen, die mit 2 bis 50 Mol Alkylenoxid pro Mol monoethylenisch ungesättigter Verbindungen modifiziert sind.

Weitere geeignete Polymere sind Polyasparaginsäure bzw. deren Derivate in nicht oder nur teilneutralisierter Form.

Besondes geeignet sind auch Pfropfpolymerisate von Acrylsäure, Methacrylsäure, Maleinsäure und weiteren ethylenisch

ungesättigten Monomeren auf Salze der Polyasparaginsäure, wie sie üblicherweise bei der zuvor beschriebenen Hydrolyse des Polysuccinimids anfallen. Hierbei kann auf die sonst notwendige Zugabe von Säure zur Herstellung der nur teilweise neutralisierten Form der Polyasparaginsäure verzichtet werden. Die Menge an Polyaspartat wird üblicherweise so gewählt, daß der Neutralisationsgrad aller im Polymerisat eingebauten Carboxylgruppen 80%, vorzugsweise 60%, nicht überschreitet.

Weitere einsetzbare Gerüststoffe sind beispielsweise die bevorzugt in Form ihrer Natriumsalze eingesetzten Percarbonsäuren, wie Zitronensäure, insbesondere Trinatriumcitrat und Trinatriumcitratdihydrat, Nitrilotriessigsäure und ihre wasserlöslichen Salze; die Alkalimetallsalze der Carboxymethyloxybernsteinsäure, Ethylendiamintetraessigsäure, Mono-, Dihydroxybernsteinsäure, α-Hydroxypropionsäure, Gluconsäure, Mellithsäure, Benzopolycarbonsäuren und wie [lacuna] in U. S. Pat. 4144226 und 4146495 offenbart.

Auch phosphathaltige Builder, beispielsweise Alkaliphosphate, die in Form ihrer alkalischen, neutralen oder sauren Natrium- oder Kaliumsalze vorliegen können, sind geeignet.

Beispiele hierfür sind Trinatriumphosphat, Tetranatriumdiphosphat, Dinatriumdihydrogenphosphat, Pentanatriumtriphosphat, sogenanntes Natriumhexametaphosphat, oligomeres Trinatriumphosphat mit Oligomerisierungsmengen im Bereich von 5 bis 1000, insbesondere 5 bis 50, sowie Gemische aus Natrium- und Kaliumsalzen.

Die Buildersubstanzen können von 5 Gew.-% bis 80 Gew.-% enthalten sein, bevorzugt ist ein Anteil von 10 Gew.-% bis 60 Gew.-%.

Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.

Prinzipiell kommen als organische Lösungsmittel alle ein- oder mehrwertigen Alkohole in Betracht. Bevorzugt werden Alkohole mit 1 bis 4 Kohlenstoffatomen, wie Methanol, Ethanol, Propanol, Isopropanol, geradkettiges und verzweigtes Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Eine vorteilhafte Mischung aus Lösungsmitteln besteht aus monomerem Alkohol, beispielsweise Ethanol und Polyethylenglykol im Verhältnis 0,5 : 1 bis 1,2 : 1.

Weitere geeignete Lösungsmittel sind beispielsweise Triacetin (Glycerintriacetat) und 1-Methoxy-2-propanol.

Als Verdickungsmittel werden bevorzugt gehärtetes Rizinusöl, Salze von langkettigen Fettsäuren, die vorzugsweise in Mengen von 0 bis 5 Gew.-% und insbesondere in Mengen von 0,5 bis 2 Gew.-% [lacuna], beispielsweise Natrium-, Kalium-, Aluminium-, Magnesium- und Titan-stearate oder die Natrium- und/oder Kaliumsalze der Behensäure, sowie Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid eingesetzt.

Als Verdicker eignen sich wasserlösliche Polyacrylate, die beispielsweise mit etwa 1 % eines Polyallylethers der Sucrose quervernetzt sind und die eine relative Molekülmasse oberhalb einer Million besitzen. Beispiele hierfür sind die unter dem Namen Carbopol® 940 und 941 erhältlichen Polymere. Die quervernetzten Polyacrylate werden in Mengen nicht über 1 Gew.-%, vorzugsweise in Mengen von 0,2 bis 0,7 Gew.-% eingesetzt.

Als Enzyme kommen solche aus der Klasse der Proteasen wie BLAP, Optimase, Opticlean, Maxacal, Maxapem, Esperase, Savinase, Purifect OxP und/ oder Durazym, Lipasen wie Lipolase, Lipomax, Lumafast und/ oder Lipozym , Amylasen wie Termamyl, Ainylase-LT, Maxamyl, Duramyl und/ oder Purafect OxAm, sowie Cutinasen, Pullulanasen bzw. deren Gemische in Frage. Ihr Anteil kann 0,2 bis 1 Gew.-% betragen. Die Enzyme können an Trägersubstanzen adsorbiert werden und/oder in Hüllsubstanzen eingebettet sein.

Als Silberkorrosionsinhibitoren können die in DE 196 49 375 genannten Verbindungen, eingesetzt werden.

Als Schaumregulatoren können vorzugsweise bis zu 6 Gew.-%,, insbesondere etwa 0.5 Gew.-% bis 4 Gew.-% schaumunterdrückende Verbindungen, vorzugsweise aus der Gruppe der Silikonöle, Gemische aus Silikonöl und hydrophobierter Kieselsäure, Paraffine, Paraffin-Alkohol-Kombinationen, hydrophobierte Kieselsäure, der Bisfettsäureamide und sonstige bekannte im Handel erhältliche Entschäumer zugesetzt werden.

Zur Einstellung eines gewünschten, sich durch die Mischung der übrigen Komponenten nicht von selbst ergebenden pH-Wertes können die erfindungsgemäßen Mittel system- und umweltverträgliche Säuren, insbesondere Zitronensäure, Essigsäure, Weinsäure, Äpfelsäure, Milchsäure, Glykolsäure, Bernsteinsäure, Glutarsäure und/ oder Adipinsäure, aber auch Mineralsäuren, insbesondere Schwefelsäure oder Alkalihydrogensulfate oder Basen, insbesondere Ammonium- oder Alkalihydroxide, enthalten. Derartige pH-Regulatoren sind in den erfindungsgemäßen Mitteln vorzugsweise nicht über 10 Gew.-%, insbesondere von 0.5 Gew.-% bis 6 Gew.-%, enthalten.

Als Konservierungsmittel eignen sich beispielsweise Phenoxyethanol, Formaldehydlösung, Pentandiol oder Sorbinsäure.

Als Perlglanzmittel kommen beispielsweise Glycoldistearinsäureester wie Ethylenglycoldistearat, aber auch Fettsäuremonoglykolester in Betracht.

Als Salze bzw. Stellmittel kommen beispielsweise Natriumsulfat, Natriumcarbonat oder Natriumsilikat (Wasserglas) in Betracht.

Als typische Einzelbeispiele für weitere Zusatzstoffe sind Natriumborat, Stärke, Saccharose, Polydextrose, RAED, Stilbenverbindungen, Methylcellulose, Toluolsulfonat, Cumolsulfonat, Seifen und Silicone zu nennen.

Die Bleichkatalysatoren dieser Erfindung können in einer Vielzahl von Produkten eingesetzt werden. Diese umfassen Textilwaschmittel, Textilbleichmittel, Oberflächenreiniger, Toilettenreiniger, Geschirrspülmaschinenreiniger und auch Gebißreiniger. Die Waschmittel können in fester oder flüssiger Form vorliegen.

Es ist aus Gründen der Stabilität und Handhabbarkeit vorteilhaft, die Bleichaktivatoren in Form von Granulaten zu verwenden, die neben dem Bleichkatalysator ein Bindemittel enthalten. Verschiedene Methoden, solche Granulate herzustellen, sind in der Patentliteratur beschrieben, so beispielsweise in Kanada Pat. Nr. 1102966, GB 1561333, US 4087369, EP 240057, EP 241962, EP 101634 und EP 62523.

Die die erfindungsgemäßen Bleichkatalysatoren enthaltenden Granulate werden im Allgemeinen der Waschmittelzusammensetzung zusammen mit den anderen, trockenen Bestandteilen wie etwa Enzymen, anorganischen Peroxidbleichmitteln zugesetzt. Die Waschmittelzusammensetzung, zu der die Katalysatorgranulate zugegeben werden, kann auf verschiedenen Wegen erhalten werden, wie etwa Mischen der trockenen Komponenten, Extrudieren oder Sprühtrocknung.

In einer weiteren Ausführungsform sind die erfindungsgemäßen Bleichkatalysatoren besonders geeignet für nicht-wässrige flüssige Waschmittel, zusammen mit einer bleichenden Peroxidverbindung wie in EP-0 869 171 beschrieben. Es handelt sich dabei um Zusammensetzungen in Form eines nichtwässrigen, flüssigen Mediums, in dem eine feste Phase dispergiert sein kann. Das nicht wässrige, flüssige Medium kann eine flüssige, oberflächenaktive Substanz sein, vorzugsweise eine nichtionische oberflächenaktive Substanz, ein nicht polares flüssiges Medium wie etwa flüssiges Paraffin, ein polares Lösungsmittel, wie etwa Polyole, zum Beispiel Glycerin, Sorbitol, Ethylenglycol, eventuell in Verbindung mit niedermolekularen einwertigen Alkoholen wie Ethanol oder Isopropanol oder Mischungen daraus.

Die feste Phase kann aus Buildersubstanzen, Alkalien, abrasiven Stoffen, Polymeren und festen ionischen oberflächenaktiven Verbindungen, Bleichmitteln, fluoreszierenden Stoffen und anderen üblichen festen Inhaltsstoffen bestehen.

Nachfolgende Beispiele sollen die Erfindung näher erläutern ohne sie darauf einzuschränken.

Beispiele

Beispiel 1: Darstellung von 2,6-Bis(4,7-dimethyl-1,4,7-triazacyclonon-1-ylmethyl)-4-methylphenol (dtmp)

Zu einer Lösung von 5 g (32 mmol) 1,4-Dimethyl-1,4,7-triazacyclononan und 3,21 g (32 mmol) Triethylamin in 150 ml Methanol wird bei 0 °C portionsweise 3,26 g (16 mmol) 2,6-Bis-chlormethyl-4-methylphenol zugegeben und anschließend 4 Tage bei Raumtemperatur gerührt. Zur Aufarbeitung werden 1,28 g (32 mmol) Natriumhydroxid zugefügt und das entstehende Kochsalz abfiltriert. Das Lösungsmittel wird i. Vak. entfernt und das Produkt ein Tag am Hochvakuum getrocknet. Ausbeute: 6,1 g (86%).
Analytik:
$^1$H-NMR: $\delta$ = 2,24 (s, 3 H, $C_{arom.}$-C$H_3$); 2,35 (s, 12 H, N-C$H_3$); 2,64 (m, 8 H, Carom.-$CH_2$-N-$CH_2$-C$H_2$-N); 2,67 (s, 8 H, $H_3$C-N-C$H_2$-C$H_2$-N-$CH_3$); 2,87 (m, 8 H, $C_{arom.}$-$CH_2$-N-C$H_2$-$CH_2$-N); 3,73 (s, 4 H, $C_{arom.}$-C$H_2$N); 6,82 (s, 2 H, $C_{arom.}$-H)
13C-NMR: $\delta$ = 20,4 (Carom.-*C*H3); 45,4 (N-CH3); 53,0; 55,9; 56,2; (N-$CH_2$-$CH_2$-N); 58,6 ($C_{arom.}$-$CH_2$-N); 123,0 ($C_{arom.}$-$CH_2$-N); 127,4 ($C_{arom.}$- $CH_3$); 130,0 ($C_{arom.}$-H), 154,7 ($C_{arom.}$-OH)
MS: (Elektrospray) m/z = 447 (M+H$^{\oplus}$, 100%)

Beispiel 2: Darstellung von Di-Mangan-$\mu$-[2,6-bis(4,7-dimethyl-1,4,7-triazacyclonon-1-ylmethyl)-4-methylphenolato]-bis ($\mu$-acetato) hexafluorophosphat (Mangan-Komplex I)

6 g (13,4 mmol) 2,6-Bis(4,7-dimethyl-1,4,7-triazacyclonon-1-ylmethyl)-4-methylphenol werden in 150 ml Methanol gelöst und innerhalb von 30 min abwechselnd in gleichen Portionen 6,6 g (27 mmol) Mangan(II)acetat · 4 $H_2O$ und 2,4 g (13,5 mmol) $KPF_6$ zugegeben. Nach einem Tag Rühren bei Raumtemperatur wird der Komplex mit Wasser ausgefällt, anschließend unter vermindertem Druck abfiltriert, mit wäßrigem Methanol gewaschen und der erhaltene grüne Feststoff zwei Tage i. Vak. getrocknet. Ausbeute: 9,9 g (91%).
Analytik:
MS: Elektrospray m/z = 673 (M - $PF_6^-$, 100%), FAB m/z = 673 (M - $PF_6^-$, 100%)

| | | | | |
|---|---|---|---|---|
| Elementaranalyse: | %N (gef.) | 10,11 | %N (calc.) | 10,24 |
| | %C (gef.) | 42,27 | %C (calc.) | 42,45 |

(fortgesetzt)

| | | | | |
|---|---|---|---|---|
| | %H (gef.) | 6,33 | %H (calc.) | 6,51 |

Die Struktur des Komplexes wurde weiterhin durch Röntgenstrukturanalyse, Cyclovoltammetrie und Magnetisierungsmessung gesichert.
Die Verbindung kristallisiert in der monoklinen Raumgruppe P 2/c mit a = 17.5505(10) Å, b = 10.3361(2) Å, c = 20,4256 (5) Å, β = 93.115(10)° und Z = 4. Die Struktur enthält positiv geladene, zweikernige Kationen, die durch das Hexafluorophosphat-Anion getrennt sind. Die Manganionen sind verbrückt durch zwei zweizähnige Acetatgruppen und den zentralen Phenoxysauerstoff des siebenzähnigen Liganden. Drei N-Donoratome bilden die Arme des Liganden, der jedes Mn-Atom facial koordiniert und die $N_3O_3$-Koordination komplettiert.
Die Cyclovoltammetrie zeigt zwei reversible Oxidationssignale bei 14 mV und 528 mV vs. Ferrocen /Ferrocinium.
Die temperaturabhängige Magnetisierungsmessung bei 1 T ergibt einen $S_{tot}$= Grundzustand und ein antiferromagnetisches Temperaturverhalten. Das effektive magnetische Moment beträgt bei 2 K 1,6 μB und steigt stetig bis auf 7.15 μB an. Die Verfeinerung ergab eine magnetische Austauschwechselwirkung von J = -4.3 $cm^{-1}$.

Beispiel 3: Darstellung von 2,6-Bis-(4,7-dimethyl-1,4,7-triazacyclonon-1-ylmethyl)-4-methylanisol (dtma)

Zu einer Lösung von 0,31 g (2 mmol) 1,4-Dimethyl-1,4,7-triazacyclononan, 0,14 g $K_2CO_3$ (1 mmol) in 10 ml Toluol werden innerhalb von 30 min 0,31 g (1 mmol) 2,6-Bis-brommethyl-4-methylanisol zugegeben und die Reaktionsmischung 2 d unter Schutzgas refluxiert.
Nach Abkühlung auf Raumtemperatur wurde das ausgefallene KBr über Celite abfiltriert und anschließend das Lösemittel unter verminderten Druck abdestilliert. Das zurückbleibende gelbliche Öl wird im Hochvakuum von restlichem Toluol befreit und bedarf keiner weiteren Reinigung. Ausbeute: 0,39 g (85%).
Analytik:
$^1$H-NMR: δ = 2.31 (s, 3 H, $C_{arom.}$-C$H_3$); 2.35 (s, 12 H, N-C$H_3$); 2.71 (m, 16 H, -$CH_2$-N-C$H_2$-C$H_2$-N-$CH_2$-); 2.93 (s, 8 H, $H_3$C-N-C$H_2$-C$H_2$-N-$CH_3$,); 3.68 (s, 4 H, $C_{arom.}$-C$H_2$N); 7.17 (s, 2 H, $C_{arom.}$-H)
$^{13}$C-NMR: δ = 21.0 ($C_{arom.}$-$CH_3$); 46.5 (N-$CH_3$); 55.8; 56.3; 57.0; 57.2 ($OCH_3$; N-$CH_2$-$CH_2$-N); 61.4 ($C_{arom.}$-$CH_2$-N); 130.3 ($C_{arom.}$-H); 132.4 ($C_{arom}$.-$CH_2$N); 132.6 ($C_{arom.}$-$CH_3$); 155.1 ($C_{arom.}$-$OCH_3$)
MS: (Elektrospray) m/z = 460 (M+H$^{\oplus}$, 100%)

Beispiel 4: Darstellung von Di-Mangan-μ-[2,6-bis-(4,7-dimethyl-1,4,7-triazacyclonon-1-ylmethyl)-4-methylanisol]-μ-oxo-bis(μ-acetato)-di-hexafluorophosphat (Mangan-Komplex II)

Zu 0,14 g (0,3 mmol) bcmmp, 0,12 g (1,5 mmol) Natriumacetat und 0,28 g (1,5 mmol) $KPF_6$ gelöst in 30 ml Methanol, werden 0,16 g (0,6 mmol) Mangan(III)acetat-trihydrat gegeben. Nach einem Tag Rühren wird der rote Feststoff im Vakuum abfiltriert, mit Methanol gewaschen und zwei Tage im Vakuum getrocknet. Erhalten wurden 0,25 g (85 %) eines rotvioletten Feststoffes.
Analytik:
MS (Elektrospray): m/z = 849 (M - $PF_6^-$, 100%)

| | | | | |
|---|---|---|---|---|
| Elementaranalyse: | %N (gef.) | 8,27 | %N (calc.) | 8,45 |
| | %C (gef.) | 35,86 | %C (calc.) | 36,23 |
| | %H (gef.) | 5,21 | %H (calc.) | 5,49 |

Die Struktur des Komplexes wurde weiterhin durch UV-VIS, IR, Spektroelektrochemie, Magnetisierungsmessung und EPR bestätigt.

Die UV-VIS-Spektren besitzen die erwarteten Banden, die Banden bei 487 und 521 nm und die zugehörigen Extinktionen sind charakteristisch für das Chromophor $Mn^{III}$-O-$Mn^{III}$:
Bande/[nm] (ε/[$lmol^{-1}cm^{-1}$]245 (5500), 283 (6200), 307 (6000), 487 (390), 521 (370), 704 (100).
Im IR ist die antisymmetrische Schwingung des verbrückenden Sauerstoffs bei 742 $cm^{-1}$ zu finden. Der Komplex ist EPR-silent.
Die Spektroelektrochemie dokumentiert die Möglichkeit der chemisch reversiblen Oxidation der $Mn^{III}Mn^{III}$-Spezies in die $Mn^{III}Mn^{IV}$- und $Mn^{III}Mn^{II}$- Formen und die charakteristische Änderung der Bandenlagen und Extinktionen. Die zugehörigen EPR-Spektren beweisen die Heterovalenz der aus dem $Mn^{III}Mn^{III}$-Komplex erzeugten Spezies. So zeigt die $Mn^{III}Mn^{IV}$-Spezies in X-Band das erwartete gut aufgelöste 16-Linien-Spektrum um g = 2.
Die Magnetisierungsmessung zeigt die schwache ferromagnetische Kopplung mit J = 5.3 $cm^{-1}$.

Bleichversuche

Durch Mischen von 200 ml einer wässrigen Lösung des Referenzwaschmittels WMP der Wäschereiforschung Krefeld (Konzentration 2 g/l WMP in Wasser mit 15° dH) mit 200 mg Natriumpercarbonat und 2 mg des jeweiligen Katalysators wurde eine Bleichmittelformulierung A hergestellt. Eine weitere Formulierung B wird durch zusätzliche Zugabe von 100 mg Tetraacetylethylendiamin (TAED) erhalten. Mit diesen Formulierungen wurden bleichempfindliche Standardtestgewebe der Wäschereiforschung Krefeld (WFK) mit den Anschmutzungen Tee (BC-1) sowie Curry (BC-4) in einem Linitest-Gerät (Heraeus) einer Behandlung bei einer Temperatur von 40 °C unter isothermen Waschbedingungen unterworfen. Nach einer dreißigminütigen Waschzeit wurden die Gewebestücke mit Wasser gespült, getrocknet und gebügelt. Anschließend wurde die Bleichwirkung durch eine Bestimmung der Differenz ΔR(Formulierung+Katalysator) der Remissionen vor und nach dem Waschvorgang mittels eines Weißgrad-Meßgerätes (ELREPHO 2000, Datacolor) quantifiziert. Aus diesen ΔR(Formulierung+Katalysator)-Werten und den in Kontrollversuchen ohne Bleichkatalysator ermittelten Werten ΔR(Formulierung) wurden die in Tabelle 1 aufgelisteten ΔΔR-Werte berechnet, die ein direktes Maß für die durch den Zusatz an Katalysator hervorgerufene Verbesserung der Bleichwirkung darstellen:

$$\Delta\Delta R = \Delta R(\text{Formulierung+Katalysator}) - \Delta R(\text{Formulierung})$$

Tabelle 1: Mittelwerte der Remissionsdifferenzen ΔΔR aus jeweils drei Bestimmungen

| Bleichformulierung | Katalysator | Tee BC-1 | Curry BC-4 |
|---|---|---|---|
| A | Mangan-Komplex I | 7,6 | 10,0 |
| B | Mangan-Komplex I | 6,0 | 8,3 |
| A | Mangan-Komplex II | 8,2 | 8,7 |
| B | Mangan-Komplex II | 4,5 | 7,0 |

Weitere vorteilhafte Eigenschaften der beschriebenen Komplexe sind geringe Farbschädigung und geringe Faserschädigung.

## Patentansprüche

**1.** Verbindungen der allgemeinen Formel I

$$[L_nM_mX_p]^zY_q \qquad \text{Formel I}$$

wobei

M für Mangan in der Oxidationsstufe II, III, IV und/oder V, Eisen in der Oxidationsstufe II und/ oder III oder Kobalt in der Oxidationsstufe II und/ oder III steht,
X eine Koordinations- oder Brückengruppe darstellt,
Y ein Gegenion in der entsprechenden stöchiometrischen Menge zum Ausgleich einer vorhandenen Ladung z bedeutet, wobei
z als Ladung des Metall-Komplexes positiv, null oder negativ sein kann,
n und m unabhängig voneinander ganze Zahlen von 1 bis 4,
p eine ganze Zahl von 0 bis 15,
q z/ Ladung von Y
L ein organischer Ligand der allgemeinen Formel II ist

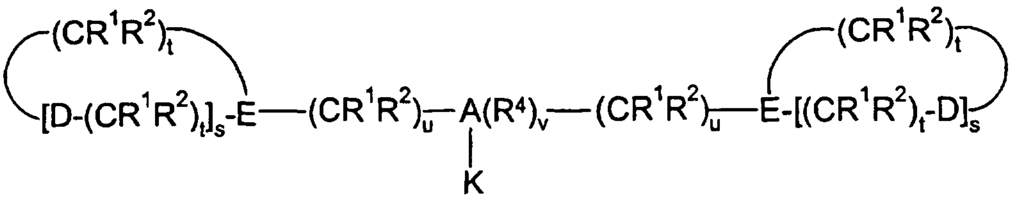

(II)

wobei

- $R^1$ und $R^2$ unabhängig voneinander Wasserstoff, eine Alkylgruppe mit 1 bis 6 Kohlenstoffatomen, eine Alkoxygruppe mit 1 bis 3 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, bedeuten,
- jedes D unabhängig voneinander $NR^3$, O, $PR^3$ oder S bedeutet, wobei $R^3$ für eine Alkylgruppe mit 1 bis 6 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, steht,
- jedes E unabhängig voneinander für N, P oder $C(R^1)$ steht, mit den oben für $R^1$ angegebenen Bedeutungen,
- t für eine Zahl von 0 bis 6, s für eine Zahl von 1 bis 5 und u für eine Zahl von 1 bis 4 stehen,
- A einen $C_3$- bis $C_9$-Cycloalkyl-, Phenyl-, 1,1'-Biphenyl-, Naphtyl-, Anthracenyl- oder Pyridinylrest bedeutet,
- jedes $R^4$ unabhängig voneinander Wasserstoff, eine $C_1$- bis $C_6$-Alkylgruppe, eine $C_1$- bis $C_3$-Alkoxygruppe, eine $C_3$- bis $C_{10}$-Cycloalkylgruppe oder eine Phenylgruppe, die auch substituiert sein kann, bedeutet,
- v für eine Zahl von 0 bis 15 steht,
- K eine an mindestens eines der Metallzentren M koordinierende Gruppe vom Typ -O, -OR, -S, -SR, $-NR_2$, -NR, $-PR_2$ oder-PR bedeutet, wobei R für Wasserstoff, eine Alkylgruppe mit 1 bis 6 Kohlenstoffatomen, eine Alkenyl- oder Alkinylgruppe mit 2 bis 8 Kohlenstoffatomen, eine Cycloalkylgruppe mit 3 bis 10 Kohlenstoffatomen oder eine Phenylgruppe, die auch substituiert sein kann, steht.

X ein Anion der folgenden Formeln bedeutet:
$F^-$, $Cl^-$, $Br^-$, $SCN^-$, $OH^-$, $O_2^{2-}$, $O^{2-}$, $O_2^-$, $R^{10}OO^-$, $H_2O$, $HS^-$, $CN^-$, $OCN^-$, $S^{2-}$, $N_3^-$, $NH_3$, $NR^{10}_3$, $NR^{10-}_2$, $R^{10}O^-$, $R^{10}COO^-$, $R^{10}SO_3^-$ und $R^{10}SO_4^-$, wobei $R^{10}$ jeweils für Wasserstoff, $C_1$- bis $C_8$-Alkyl, $C_3$- bis $C_8$-Cycloalkyl oder $C_6$- bis $C_{18}$-Aryl steht, und
Y - bei positivem z ein Anion der folgenden Formeln: $F^-$, $Cl^-$, $Br^-$, $NO_3^-$, $ClO_4^-$, $SCN^-$, $PF_6^-$, $R^{10}SO_4^-$, $R^{10}COO^-$, $R^{10}SO_3^-$,$BF_4^-$, $BPh_4^-$ und $SO_4^{2-}$und
- bei negativem z ein Kation der folgenden Formeln: $Li^+$, $Na^+$, $K^+$, $Mg^{2+}$, $Ca^{2+}$, $Al^{3+}$, $NH_4^+$, $R^{10}NH_3^+$, $R^{10}_2NH_2^+$, $R^{10}_3NH^+$ und $R^{10}_4N^+$, wobei $R^{10}$ die zuvor genannte Bedeutung hat, bedeutet.

2. Verbindungen der allgemeinen Formel I nach Anspruch 1, **dadurch gekennzeichnet, daß** D für $NR^3$, E für Stickstoff, $R^1$ und $R^2$ für Wasserstoff, s für eine Zahl von 1 bis 3, t für eine Zahl von 1 bis 4 und K für eine Gruppe vom Typ -O, -OR, -S oder $-NR_2$ stehen und die übrigen Symbole die in Anspruch 1 genannten Bedeutungen haben.

3. Verbindungen der allgemeinen Formel I nach Anspruch 1, **dadurch gekennzeichnet, daß** D für $NR^3$, E für Stickstoff, $R^1$ und $R^2$ für Wasserstoff, s für eine Zahl von 1 bis 3, t für eine Zahl von 1 bis 4, A für eine Phenyl- oder $C_3$-$C_9$-Cycloalkylgruppe, m für eine Zahl von 1 bis 3, n für eine Zahl von 1 bis 2 und K für eine Gruppe vom Typ -O, -OR, -S oder $-NR_2$ stehen und die übrigen Symbole die in Anspruch 1 genannten Bedeutungen haben.

4. Verbindungen der Formel I, gemäß Anspruch 1, **dadurch gekennzeichnet, dass** der Ligand L für 2,6-Bis(4,7-dimethyl-1,4,7-triazacyclonon-1-ylmethyl)-4-methyl-phenolat steht.

5. Verbindungen der Formel I, gemäß Anspruch 1, **dadurch gekennzeichnet, dass** der Ligand L für 2,6-Bis(4,7-dimethyl-1,4,7-triazacyclonon-1-ylmethyl)-4-methylanisol steht.

6. Verbindungen der Formel I, gemäß Anspruch 1, **dadurch gekennzeichnet, dass** das Übergangsmetall M Mangan ist.

7. Verwendung der Verbindungen der Formel I gemäß einem oder mehreren der Ansprüche 1 bis 6 als Bleichkatalysatoren in Peroxi-Verbindungen enthaltenden Wasch-, Reinigungs- und Desinfektionsmitteln sowie bei der Textil- und Papierbleiche.

**8.** Verwendung der Verbindungen der Formel I gemäß Anspruch 7, **dadurch gekennzeichnet, dass** das Wasch-, Reinigungs- und Desinfektionsmittel ein Maschinengeschirrspülmittel oder eine Waschmittelformulierung ist.

## Claims

**1.** A compound of the general formula I

$$[L_nM_mX_p]^zY_q \qquad \text{formula I}$$

where

M is manganese in oxidation state II, III, IV and/or V, iron in oxidation state II and/or III or cobalt in oxidation state II and/or III,
X is a coordination group or bridging group,
Y is a counterion in the corresponding stoichiometric amount to balance an existing charge z, where
z as the charge of the metal complex can be positive, zero or negative,
n and m, independently of one another, are integers from 1 to 4,
p is an integer from 0 to 15,
q is z/charge of Y,
L is an organic ligand of the general formula II

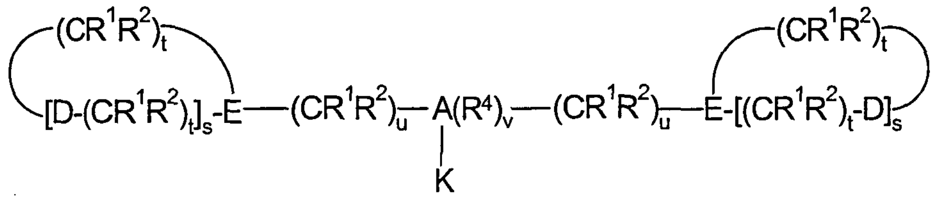

(II)

where

- $R^1$ and $R^2$, independently of one another, are hydrogen, an alkyl group having 1 to 6 carbon atoms, an alkoxy group having 1 to 3 carbon atoms, an alkenyl or alkinyl group having 2 to 8 carbon atoms, a cycloalkyl group having 3 to 10 carbon atoms or a phenyl group which may also be substituted,
- each D, independently of one another, is $NR^3$, O, $PR^3$ or S, where $R^3$ is an alkyl group having 1 to 6 carbon atoms, an alkenyl or alkinyl group having 2 to 8 carbon atoms, a cycloalkyl group having 3 to 10 carbon atoms or a phenyl group which may also be substituted,
- each E, independently of one another, is N, P or $C(R^1)$ with the meanings given above for $R^1$,
- t is a number from 0 to 6, s is a number from 1 to 5 and u is a number from 1 to 4,
- A is a $C_3$- to $C_9$-cycloalkyl, phenyl, 1,1'-biphenyl, naphthyl, anthracenyl or pyridinyl radical,
- each $R^4$, independently of one another, is hydrogen, a $C_1$- to $C_6$- alkyl group, a $C_1$- to $C_3$-alkoxy group, a $C_3$- to $C_{10}$-cycloalkyl group or a phenyl group which may also be substituted,
- v is a number from 0 to 15,
- K is a group coordinating to at least one of the metal centers M and of the type -O, -OR, -S, -SR, $-NR_2$, -NR, $-PR_2$ or -PR, where R is hydrogen, an alkyl group having 1 to 6 carbon atoms, an alkenyl or alkinyl group having 2 to 8 carbon atoms, a cycloalkyl group having 3 to 10 carbon atoms or a phenyl group which may also be substituted,

X is an anion of the following formulae:
$F^-$, $Cl^-$, $Br^-$, $SCN^-$, $OH^-$, $O_2^{2-}$, $O^{2-}$, $O_2^-$, $R^{10}OO^-$, $H_2O$, $HS^-$, $CN^-$, $OCN^-$, $S^{2-}$, $N_3^-$, $NH_3$, $NR^{10}_3$, $NR^{10}_2{}^-$, $R^{10}O^-$, $R^{10}COO^-$, $R^{10}SO_3^-$ and $R^{10}SO_4^-$, where $R^{10}$ is in each case hydrogen, $C_1$- to $C_8$-alkyl, $C_3$- to $C_8$-cycloalkyl or $C_6$- to $C_{18}$-aryl, and
Y - if z is positive, is an anion of the following formulae: $F^-$, $Cl^-$, Br , $NO_3^-$, $ClO_4^-$, $SCN^-$, $PF_6^-$, $R^{10}SO_4^-$, $R^{10}COO^-$,

$R^{10}SO_3^-$, $BF_4^-$, $BPh_4^-$ and $SO_4^{2-}$ and
- if z is negative, is a cation of the following formulae: $Li^+$, $Na^+$, $K^+$, $Mg^{2+}$, $Ca^{2+}$, $Al^{3+}$, $NH_4^+$, $R^{10}NH_3^+$, $R^{10}{}_2NH_2^+$, $R^{10}{}_3NH^+$ and $R^{10}{}_4N^+$, where $R^{10}$ has the meaning given above.

**2.** A compound of the formula I as claimed in claim 1, wherein D is $NR^3$, E is nitrogen, $R^1$ and $R^2$ are hydrogen, s is a number from 1 to 3, t is a number from 1 to 4 and K is a group of the type -O, -OR, -S or $-NR_2$, and the other symbols have the meanings given in claim 1.

**3.** A compound of the formula I as claimed in claim 1, wherein D is $NR^3$, E is nitrogen, $R^1$ and $R^2$ are hydrogen, s is a number from 1 to 3, t is a number from 1 to 4, A is a phenyl or $C_3$-$C_9$-cycloalkyl group, m is a number from 1 to 3, n is a number from 1 to 2 and K is a group of the type -O, -OR, -S or $-NR_2$, and the other symbols have the meanings given in claim 1.

**4.** A compound of the formula I as claimed in claim 1, wherein the ligand L is 2,6-bis(4,7-dimethyl-1,4,7-triazacyclonon-1-ylmethyl)-4-methylphenoxide.

**5.** A compound of the formula I as claimed in claim 1, wherein the ligand L is 2,6-bis(4,7-dimethyl-1,4,7-triazacyclonon-1-ylmethyl)-4-methylanisole.

**6.** A compound of the formula I as claimed in claim 1, wherein the transition metal M is manganese.

**7.** The use of the compounds of the formula I as claimed in one or more of claims 1 to 6 as bleaching catalysts in detergents, cleaners and disinfectants which comprise peroxy compounds, and in textile and paper bleaching.

**8.** The use of the compounds of the formula I as claimed in claim 7, wherein the detergent, cleaner or disinfectant is a dishwasher detergent or a detergent formulation.

## Revendications

**1.** Composés de formule générale I

$$[L_nM_mX_p]^zY_q \qquad \text{Formule I}$$

dans laquelle

M représente le manganèse à l'état d'oxydation II, III, IV et/ou V, le fer à l'état d'oxydation II et/ou III ou le cobalt à l'état d'oxydation II et/ou III,
X représente un groupe de coordination ou pontant,
Y représente un contre-ion dans la quantité stoechiométrique correspondante pour égaler la charge z présente, dans laquelle
z peut être comme charge du complexe métallique positif, nul ou négatif,
n et m représentent indépendamment l'un de l'autre des nombres entiers de 1 à 4,
p est un nombre entier de 0 à 15,
q représente z/charge de Y
L est un ligand organique de formule générale II

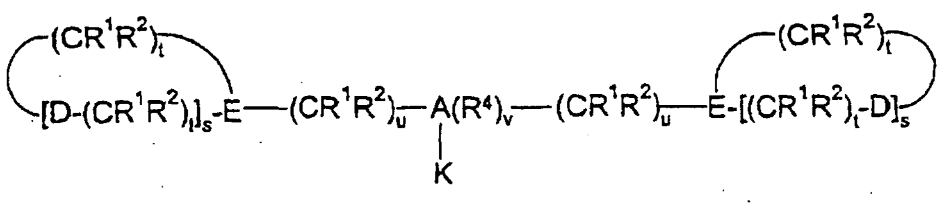

(II)

dans laquelle

- $R^1$ et $R^2$ représentent indépendamment l'un de l'autre un hydrogène, un groupe alkyle avec 1 à 6 atomes de carbone, un groupe alcoxy avec 1 à 3 atomes de carbone, un groupe alcényle ou alcynyle avec 2 à 8 atomes de carbone, un groupe cycloalkyle avec 3 à 10 atomes de carbone ou un groupe phényle, qui peut aussi être substitué,
- chaque D représente indépendamment des autres $NR^3$, O, $PR^3$ ou S, dans lequel $R^3$ représente un groupe alkyle avec 1 à 6 atomes de carbone, un groupe alcényle ou alcynyle avec 2 à 8 atomes de carbone, un groupe cycloalkyle avec 3 à 10 atomes de carbone ou un groupe phényle, qui peut aussi être substitué,
- chaque E représente indépendamment de l'autre N, P ou $C(R^1)$, avec les significations indiquées ci-dessus pour $R^1$,
- t représente un nombre de 0 à 6, s un nombre de 1 à 5 et u un nombre de 1 à 4,
- A représente un radical cycloalkyle en $C_3$ à $C_9$, phényle, 1,1'-biphényle, naphtyle, anthracényle ou pyridinyle,
- chaque $R^4$ représente indépendamment des autres un hydrogène, un groupe alkyle en $C_1$ à $C_6$, un groupe alcoxy en $C_1$ à $C_3$, un groupe cycloalkyle en $C_3$ à $C_{10}$ ou un groupe phényle, qui peut aussi être substitué,
- v représente un nombre de 0 à 15,
- K représente un groupe coordinant au moins un des centres métalliques M de type -O, -OR, -S, -SR, $-NR_2$, -NR, $-PR_2$ ou -PR, R représentant un hydrogène, un groupe alkyle avec 1 à 6 atomes de carbone, un groupe alcényle ou alcynyle avec 2 à 8 atomes de carbone, un groupe cycloalkyle avec 3 à 10 atomes de carbone ou un groupe phényle, qui peut aussi être substitué,

X représente un anion des formules suivantes : $F^-$, $Cl^-$, $Br^-$, $SCN^-$, $OH^-$, $O_2^{2-}$, $O^{2-}$, $O_2^-$, $R^{10}OO^-$, $H_2O$, $HS^-$, $CN^-$, $OCN^-$, $S^{2-}$, $N_3^-$' $NH_3$, $NR^{10}{}_3$, $NR^{10}{}_2^-$, $R^{10}O^-$, $R^{10}COO^-$, $R^{10}SO_3^-$ et $R^{10}SO_4^-$, dans lesquelles $R^{10}$ représente respectivement un hydrogène, alkyle en $C_1$ à $C_8$, cycloalkyle en $C_3$ à $C_8$, ou aryle en $C_6$ à $C_{18}$, et Y -pour z positif représente un anion des formules suivantes : $F^-$, $Cl^-$, $Br^-$, $NO_3^-$, $ClO_4^-$' $SCN^-$, $PF_6^-$, $R^{10}SO_4$ $R^{10}COO^-$, $R^{10}SO_3^-$, $BF_4^-$, $BPh_4^-$ et $SO_4^{2-}$ et -pour z négatif un cation des formules suivantes : $Li^+$, $Na^+$, $K^+$, $Mg^{2+}$, $Ca^{2+}$, $Al^{3+}$, $NH_4^+$, $R^{10}NH_3^+$, $R^{10}{}_2NH_2^+$, $R^{10}{}_3NH^+$ et $R^{10}{}_4N^+$, dans lesquelles $R^{10}$ a la signification indiquée précédemment.

2. Composés de formule générale I selon la revendication 1, **caractérisés en ce que** D représente $NR^3$, E un azote, $R^1$ et $R^2$ un hydrogène, s un nombre de 1 à 3, t un nombre de 1 à 4 et K un groupe du type -O, -OR, -S ou $-NR_2$ et les symboles usuels ont les significations indiquées à la revendication 1.

3. Composés de formule générale I selon la revendication 1, **caractérisés en ce que** D représente $NR^3$, E représente un azote, $R^1$ et $R^2$ un hydrogène, s un nombre de 1 à 3, t un nombre de 1 à 4, A un groupe phényle ou cycloalkyle en $C_3$ à $C_9$, m un nombre de 1 à 3, n un nombre de 1 à 2 et K un groupe du type -O, -OR, -S ou $-NR_2$ et les symboles usuels ont les significations indiquées à la revendication 1.

4. Composés de formule I selon la revendication 1, **caractérisés en ce que** le ligand L représente le 2,6-bis(4,7-diméthyl-1,4,7-triazacyclonon-1-ylméthyl)-4-méthyl-phénolate.

5. Composés de formule I, selon la revendication 1, **caractérisés en ce que** le ligand L représente le 2,6-bis(4,7-diméthyl-1,4,7-triazacyclonon-1-ylméthyl)-4-méthylanisol.

6. Composés de formule I selon la revendication 1, **caractérisés en ce que** le métal de transition M est le manganèse.

7. Utilisation des composés de formule I selon une ou plusieurs des revendications 1 à 6 comme catalyseurs de blanchiment dans des lessives, détergents et désinfectants contenant des composés peroxyde ainsi que dans le blanchiment du textile et du papier.

8. Utilisation des composés de formule I selon la revendication 7, **caractérisée en ce que** la lessive, le détergent et le désinfectant est un agent de rinçage de lave-vaisselle ou une formulation de lessive.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9606154 A
- EP 458397 A
- DE 19616693
- DE 19616767
- EP 0525239 A
- DE 19529905
- DE 19605688
- GB 1082179 A
- US 3929678 A
- US 4144226 A
- US 4146495 A
- DE 19649375
- CA 1102966
- GB 1561333 A
- US 4087369 A
- EP 240057 A
- EP 241962 A
- EP 101634 A
- EP 62523 A
- EP 0869171 A

### In der Beschreibung aufgeführte Nicht-Patentliteratur

- *J. Am. Chem. Soc.,* 1989, vol. 111, 5102-5114
- *J. Am. Chem. Soc.,* 1994, vol. 116 (22), 10334-10335